# EUROPÄISCHE PATENTSCHRIFT

(11) **EP 1 937 714 B1**
(45) Veröffentlichungstag und Bekanntmachung des Hinweises auf die Patenterteilung: **14.08.2013**
(21) Anmeldenummer: 06792342.5
(22) Anmeldetag: 30.09.2006
(51) Int. Cl.: C07K 14/435, A61P 9/10

(54) **EGLN2-VARIANTEN UND IHRE VERWENDUNG BEI DER VORBEUGUNG ODER BEHANDLUNG THROMBOEMBOLISCHER ERKRANKUNGEN UND KORONARER HERZERKRANKUNGEN**
EGLN2 VARIANTS AND USE THEREOF IN PREVENTING OR TREATING THROMBOEMBOLIC DISORDERS AND CORONARY HEART DISEASES
VARIANTES D'EGLN2 ET LEUR UTILISATION DANS LA PROPHYLAXIE OU LE TRAITEMENT DE MALADIES THROMBOEMBOLIQUES ET DE MALADIES CARDIAQUES CORONARIENNES

(30) Priorität: 12.10.2005 DE 102005048898
(43) Veröffentlichungstag der Anmeldung: 02.07.2008
(73) Patentinhaber: SANOFI, 75008 Paris (FR)
(72) Erfinder: KOZIAN, Detlef, 65926 Frankfurt am Main (DE); HERRMANN, Matthias, 65926 Frankfurt am Main (DE)
(74) Vertreter: Schneider, Rudolf
(86) Internationale Anmeldenummer: PCT/EP2006/009518
(87) Internationale Veröffentlichungsnummer: WO 2007/042166

(56) Entgegenhaltungen:
- WO-A-2004/058052
- KENNETH K W TO AND L ERIC HUANG: "SUPPRESSION OF HYPOXIA-INDUCIBLE FACTOR 1 (HIF-1) TRANSCRIPTIONAL ACTIVITY BY THE HIF PROLYL HYDROXYLASE EGLN1" JOURNAL OF BIOLOGICAL CHEMISTRY, THE AMERICAN SOCIETY OF BIOLOGICAL CHEMISTS, INC.,, US, Bd. 280, Nr. 45, 2005, Seiten 28102-38107, XP009082562 ISSN: 0021-9258
- TUOMAINEN TOMI-PEKKA ET AL: "INCREASED RISK OF ACUTE MYOCARDIAL INFARCTION IN CARRIERS OF THE HEMOCHROMATOSIS GENE CYS282TYR MUTATION. A PROSPECTIVE COHORT STUDY IN MEN IN EASTERN FINLAND" CIRCULATION, AMERICAN HEART ASSOCIATION, DALLAS, TX, US, Bd. 100, 21. September 1999 (1999-09-21), Seiten 1274-1279, XP009082573 ISSN: 0009-7322
- TAKAGI S ET AL: "A GPVI POLYMORPHISM IS A RISK FACTOR FOR MYOCARDIAL INFARCTION IN JAPANESE" ATHEROSCLEROSIS, AMSTERDAM, NL, Bd. 165, 2002, Seiten 397-398, XP009082574 ISSN: 0021-9150

## Beschreibung

Die vorliegende Erfindung betrifft menschliche EGLN2-Varianten mit einem Serin oder einem Leucin in Position 58 der Aminosäuresequenz und deren Verwendung bei zur der Prognose thromboembolischer Erkrankungen oder koronarer Herzerkrankungen, insbesondere von Schlaganfall, prolongiertem reversiblem ischämischem neurologischem Defizit (PRIND), transitorischer ischämischer Attacke (TIA), Myokardinfarkt und/oder frühzeitigem Myokardinfarkt.

EGLN2, das aufgrund seiner HIF-Prolylhydroxylaseaktivität auch unter dem Namen Prolylhydroxylasedomäne-haltiges Protein 1 (PHD1) bekannt ist, gehört zu einer Gruppe nahe verwandter Proteine der Egl-Nine-Genfamilie, die eine aus fünf codierenden Exons bestehende konservierte genomische Struktur aufweist. Bei HIF (Hypoxie-induzierbarer Faktor) handelt es sich um einen Transkriptionsregulator, dem in vielen Aspekten der Sauerstoffhomöostase eine Schlüsselrolle zukommt, wobei jedoch der Beitrag der EGLN-Isoformen EGLN1 (PHD1), EGLN2 (PHD2) und EGLN3 (PHD3) zur physiologischen Regulation von HIF noch ungewiß ist (Appelhoff, R. J. et al. (2004) J. Biol. Chem., 279, 38458-38465, Nr. 37). Es liegen Berichte vor, nach denen alle EGLN-Isoformen ein unterschiedliches zellspezifisches und induzierbares Verhalten zeigen, was Flexibilität bei der Regulation der HIF-Reaktion auf Hypoxie gestatten sollte. Dies würde bedeuten, daß die spezifische pharmakologische Hemmung eines bestimmten EGLN-Isoenzyms das Potential zur selektiven Modulation der HIF-Reaktion besitzen könnte, die bei therapeutischen Anwendungen von Nutzen wäre (Appelhoff, R. J. et al. (2004), supra). Die Hemmung von EGLN2 sollte beispielsweise unter Ruhebedingungen die HIF-Reaktion breit über eine Reihe von Zellarten aktivieren. Im Gegensatz dazu sollte durch spezifische Hemmung von EGLN3 die Reaktion auf Hypoxie in bestimmten Geweben, die hohe Expressionsniveaus des Enzyms aufweisen, verstärkt werden (Appelhoff, R. J. et al. (2004), supra). Dadurch könnte eine Behandlung ischämischer/hypoxischer Erkrankungen ermöglicht werden. Im Gegensatz zu EGLN2 und EGLN3 ist über die physiologische Rolle von EGLN1 nur wenig bekannt.

Zum besseren Verständnis einer möglichen Beteiligung von EGLN2 an Auftreten und Verlauf koronarer Herzerkrankungen wurden bei einer hinsichtlich einer Variation im EGLN2-Gen in Position 470 der unter der Referenznummer NM_053046.2 gemäß der vorliegenden Erfindung veröffentlichten EGLN2-Referenzsequenz gut charakterisierten Patientengruppe Genotyp/Phänotyp-Assoziationsanalysen durchgeführt. Unterschiedliche genetische Varianten des EGLN2-Gens sind bereits als SNPs (Single Nucleotide Polymorphisms [= Einzelnukleotidpolymorphismen]) bekannt und veröffentlicht unter http://www.ncbi.nlm.nih.gov/SNP/snp ref.cqi?locusld=112398).

Überraschenderweise stellte sich nun heraus, daß eine Variation des Nukleotids in Position 470, insbesondere von Cytidin zu Thymidin einer für das menschliche EGLN2-Protein codierenden Nukleinsäure oder der Aminosäure in Position 58, insbesondere von Serin zu Leucin des menschlichen EGLN2-Proteins, mit dem Auftreten thromboembolischer Erkrankungen und/oder koronarer Herzerkrankungen korrehert.

Daher betrifft ein Gegenstand der vorliegenden Erfindung das EGLN2-Protein mit einer Aminosäuresequenz gemäß SEQ ID NO: 3 sowie eine für das EGLN2-Protein codierende Nukleinsäure, insbesondere eine Nukleinsäuresequenz gemäß SEQ ID NO: 4. Vorzugsweise handelt es sich bei den Nukleinsäuren gemäß der vorliegenden Erfindung um DNA oder RNA, vorzugsweise eine DNA, insbesondere eine Doppelstrang-DNA. Die Sequenz der Nukleinsäuren läßt sich weiterhin dadurch charakterisieren, daß sie mindestens ein Intron und/oder eine PolyA-Sequenz aufweist.

In einer weiteren erfindungsgemäßen Ausführungsform können die Nukleinsäuren zur Herstellung eines Vektors, vorzugsweise in Form eines Shuttle-Vektors, Phagemids, Cosmids, Expressionsvektors oder eines Vektors mit gentherapeutischer Aktivität, verwendet werden. Weiterhin lassen sich mit den oben beschriebenen Nukleinsäuren Knock-out-Genkonstrukte oder Expressionskassetten herstellen. Bei dem Expressionsvektor kann es sich um einen prokaryontischen oder einen eukaryontischen Expressionsvektor handeln. Beispiele für prokaryontische Expressionsvektoren sind beispielsweise die Vektoren pGEM oder pUC-Derivate zur Expression in *E.coli*; Beispiele für eukaryontische Expressionsvektoren sind beispielsweise die Vektoren p426Met25 or p426GAL1 (Mumberg et al. (1994) Nucl. Acids Res., 22, 5767-5768) zur Expression in *Saccharomyces cerevisiae*, *Baculovirus-*Vektoren, wie z.B. die in der EP-B1-0 127 839 oder der EP-B1-0 549 721 offenbarten, zur Expression in Insektenzellen und die Vektoren Rc/CMV und Rc/RSV oder SV40-Vektoren zur Expression in Säugerzellen, wobei alle diese Vektoren allgemein erhältlich sind. Im allgemeinen enthalten die Expressionsvektoren auch für die jeweilige Wirtszelle geeignete Promotoren, wie beispielsweise den trp-Promotor zur Expression in E.coli (siehe beispielsweise EP-B1-0 154 133), die Promotoren Met 25, GAL 1 bzw. ADH2 zur Expression in Hefen (Russel et al. (1983), J. Biol. Chem. 258, 2674-2682; Mumberg, supra), den Baculovirus-Polyhedrinpromotor zur Expression in Insektenzellen (siehe beispielsweise EP-B1-0 127 839). Zur Expression in Säugerzellen eignen sich beispielsweise solche Promotoren, die eine konstitutive, regulierbare, gewebespezifische oder stoffwechselspezifische Expression in eukaryontischen Zellen gestatten. Bei regulierbaren Elementen gemäß der vorliegenden Erfindung handelt es sich um Promotoren, Aktivatorsequenzen, Enhancer, Silencer und/oder Repressorsequenzen. Geeignete regulierbare Elemente, die eine konstitutive Expression in Eukaryonten ermöglichen, sind beispielsweise Promotoren, die von der RNA-Polymerase III erkannt werden, oder virale Promotoren, CMV-Enhancer, CMV-Promotor (siehe auch Beispiel 13), SV40-Promotor oder LTR-Promotoren, z.B. von MMTV (Mouse Mammary Tumour Virus; Lee et al. (1981) Nature 214, 228-232) sowie weitere virale Promotor und Aktivatorsequenzen, die beispielsweise aus HBV, HCV, HSV, HPV, EBV, HTLV oder HIV stammen. Zu den regulierbaren Elementen, die eine induzierbare Expression in Eukaryonten ermöglichen, gehört beispielsweise der Tetracyclin-Operator in Kombination mit einem entsprechenden Repressor (Gossen M. et al. (1994) Curr. Opin. Biotechnol. 5, 516-20). Beispiele für regulierbare Elemente, die eine stoffwechselspezifische Expression in Eukaryonten ermöglichen, sind Promotoren, die vorzugsweise durch Hypoxie reguliert werden.

Um die Einführung von gemäß der vorliegenden Erfindung verwendeten Nukleinsäuren und somit die Expression des Polypeptids in einer eu- oder prokaryontischen Zelle über eine Transfektion, Transformation oder Infektion zu ermöglichen, kann die Nukleinsäure in Form eines Plasmids, als Teil eines viralen oder nichtviralen Vektors vorliegen. Als virale Vektoren eignen sich hier insbesondere Baculoviren, Vacciniaviren, Adenoviren, adeno-assoziierte Viren sowie Herpesviren. Als nichtvirale Vektoren eignen sich hier insbesondere Virosomen, Liposomen, kationische Lipide oder polylysinkonjugierte DNA.

Vektoren mit gentherapeutischer Aktivität sind beispielsweise Virusvektoren, zum Beispiel Adenovirusvektoren oder retrovirale Vektoren (Lindemann et al., 1997, Mol. Med. 3: 466-76; Springer et al., 1998, Mol. Cell. 2: 549-58). Eukaryontische Expressionsvektoren eignen sich in isolierter Form zum gentherapeutischen Einsatz, da nackte DNA nach topischer Applikation in Hautzellen eindringen kann (Hengge et al., 1996, J. Clin. Invest. 97: 2911-6; Yu et al., 1999, J. Invest. Dermatol. 112: 370-5). Vektoren mit gentherapeutischer Aktivität lassen sich auch durch Komplexieren der gemäß der vorliegenden Erfindung verwendeten Nukleinsäure mit Liposomen erhalten, da sich auf diese Weise eine sehr hohe Transfektionseffizienz, insbesondere von Hautzellen, erzielen läßt (Alexander und Akhurst, 1995, Hum. Mol. Genet. 4: 2279-85). Bei der Lipofektion werden durch Ultraschallbehandlung der Liposomensuspension aus kationischen Lipiden kleinewnilamellare Vesikel hergestellt. Die DNA wird ionisch an die Oberfläche der Liposomen gebunden, und zwar in einem solchen Verhältnis, daß eine positive Nettoladung erhalten bleibt und die Plasmid-DNA mit 100% der Liposomen komplexiert wird. Zusätzlich zu den von Felgner et al. (1987, supra) eingesetzten Lipidgemischen DOTMA (1,2-Dioleyloxypropyl-3-trimethylammonium-bromid) und DPOE (Dioleoylphosphatidylethanolamin) wurden mittlerweile zahlreiche neuartige Lipidformulierungen synthetisiert und auf ihre Effizienz bei der Transfektion verschiedener Zellinien getestet (Behr, J.P. et al. (1989), Proc. Natl. Acad. Sci. USA 86, 6982-6986; Felgner, J.H. et al. (1994) J. Biol. Chem. 269, 2550-2561; Gao, X. & Huang, L. (1991), Biochim. Biophys. Acta 1189, 195-203). Beispiele für Lipidformulierungen sind DOTAP N-[1-(2,3-Dioleoyloxy)propyl]-N,N,N-trimethylammoniumethylsulfat oder DOGS (TRANSFECTAM; Dioctadecylamidoglycylspermin). Bei den die Übertragung von Nukleinsäuren in die Zelle erhöhenden Hilfsstoffen kann es sich beispielsweise um an DNA gebundene Proteine oder Peptide oder um synthetische Peptid-DNA-Moleküle handeln, die den Transport der Nukleinsäure in den Zellkern ermöglichen (Schwartz et al. (1999) Gene Therapy 6, 282; Brandén et al. (1999) Nature Biotech. 17, 784). Zu den Hilfsstoffen gehören auch Moleküle, die die Freisetzung von Nukleinsäuren in das Zytoplasma der Zelle ermöglichen (Planck et al. (1994) J. Biol. Chem. 269, 12918; Kichler, et al. (1997) Bioconj. Chem. 8, 213), oder beispielsweise Liposomen (Uhlmann und Peymann (1990) supra). Eine weitere besonders geeignete Form von gentherapeutischen Vektoren läßt sich durch Aufbringen der gemäß der vorliegenden Erfindung verwendeten Nukleinsäure auf Goldpartikel und das Einführen der Letzteren in Gewebe, vorzugsweise in die Haut, oder Zellen durch Beschießen mit Hilfe der sogenannten Genkanone erhalten (Beispiel 13; Wang et al., 1999, J. Invest. Dermatol., 112: 775-81, Tuting et al., 1998, J. Invest. Dermatol. 111: 183-8).

Zur gentherapeutischen Verwendung der oben beschriebenen Nukleinsäure ist es auch vorteilhaft, wenn der für das Polypeptid codierende Teil der Nukleinsäure eine oder mehrere nichtcodierende Sequenzen, einschließlich Intronsequenzen, vorzugsweise zwischen Promotor und dem Startcodon des Polypeptids, und/oder eine PolyA-Sequenz, insbesondere die natürlich vorkommende PolyA-Sequenz oder eine SV40-Virus-PolyA-Sequenz, vor allem am 3'-Ende des Gens enthält, da sich dadurch eine Stabilisierung der mRNA erreichen läßt (Jackson, R.J. (1993) Cell 74, 9-14 und Palmiter, R.D. et al. (1991) Proc. Natl. Acad. Sci. USA 88, 478-482).

Knock-out-Genkonstrukte sind dem Fachmann beispielsweise aus den US-Patenten 5,625,122; US 5,698,765; US 5,583,278 und US 5,750,825 bekannt.

Die vorliegende Erfindung betrifft ferner eine Wirtszelle, die mit einem erfindungsgemäßen Vektor oder einem Knock-out-Genkonstrukt transformiert wird. Bei den Wirtszellen kann es sich entweder um prokaryontische oder eukaryontische Zellen handeln, wobei prokaryontische Wirtszellen beispielsweise *E.coli* und eukaryontische Zellen beispielsweise *Saccharomyces cerevisiae* oder Insektenzellen sind. Als transformierte Wirtszelle besonders bevorzugt ist eine transgene embryonale nichtmenschliche Stammzelle, die dadurch gekennzeichnet ist, daß sie ein erfindungsgemäßes Knock-out-Genkonstrukt oder eine erfindungsgemäße Expressionskassette umfaßt. Verfahren zur Transformation von Wirtszellen und/oder Stammzellen sind dem Fachmann allgemein bekannt und umfassen beispielsweise die Elektroporation oder die Mikroinjektion.

Ein weiterer Gegenstand der vorliegenden Erfindung betrifft ein eine Nukleinsäure oder einen Vektor gemäß der vorliegenden Erfindung enthaltendes transgenes Tier.

Verfahren zur Herstellung nicht-hunicht transgener Tiere, insbesondere der Maus, sind dem Fachmann gleichfalls aus der DE 196 25 049 sowie der US 4,736,866, US 5,625,122, US 5,698,765, US 5,583,278 und US 5,750,825 bekannt und beinhalten transgene Tiere, die sich beispielsweise mittels direkter Injektion von Expressionsvektoren (siehe oben) in Embryos oder Spermatozyten oder mittels Transfektion von Expressionsvektoren in embryonale Stammzellen produzieren lassen (Polites und Pinkert: DNA Microinjection and Transgenic Animal Production, Seite 15 bis 68 in Pinkert, 1994: Transgenic animal technology: a laboratory handbook, Academic Press, London, UK; Houdebine, 1997, Harwood Academic Publishers, Amsterdam, Niederlande; Doetschman: Gene Transfer in Embryonic Stem Cells, Seite 115 bis 146 in Pinkert, 1994, supra; Wood: Retrovirus-Mediated Gene Transfer, Seite 147 bis 176 in Pinkert, 1994, supra; Monastersky: Gene Transfer Technology; Alternative Techniques and Applications, Seite 177 bis 220 in Pinkert, 1994, supra). Werden erfindungsgemäß verwendete Nukleinsäuren in sogenannte Targeting-Vektoren (Pinkert, 1994, supra) integriert, so besteht die Möglichkeit, nach Transfektion embryonaler Stammzellen und homologer Rekombination beispielsweise Knock-out-Mäuse zu erzeugen, die als heterozygote Mäuse im allgemeinen eine reduzierte Expression der Nukleinsäure zeigen, während homozygote Mäuse keine Expression der Nukleinsäure mehr zeigen. Auf diese Weise produzierte transgene und Knock-out-Zellen oder -Tiere lassen sich auch zum Screening und zur Identifizierung pharmakologischer Wirkstoffvektoren mit gentherapeutischer Aktivität verwenden.

Ein weiterer Gegenstand der vorliegenden Erfindung betrifft einen spezifisch an das EGLN2-Protein mit einer Aminosäuresequenz gemäß SEQ ID NO: 3 bindenden Antikörper.

Gemäß der vorliegenden Erfindung wird unter dem Begriff "spezifisch" verstanden, daß der Antikörper an das EGLN2-Protein mit einer Aminosäuresequenz gemäß SEQ ID NO: 3, jedoch im wesentlichen nicht an das EGLN2-Protein mit einer Aminosäuresequenz gemäß SEQ ID NO: 2 bindet, d.h. der Antikörper eignet sich zur Differenzierung zwischen einem EGLN2-Protein mit einem Serin in Position 58 und einem EGLN2-Protein mit einem Leucin in Position 58.

Bei diesem Antikörper handelt es sich entweder um einen polyklonalen oder einen monoklonalen, vorzugsweise einen monoklonalen Antikörper. Unter dem Begriff Antikörper werden gemäß der vorliegenden Erfindung auch Antikörper oder Antigen bindende Teile davon verstanden, die gentechnisch hergestellt und gegebenenfalls modifiziert wurden, wie beispielsweise chimärische Antikörper, humanisierte Antikörper, multifunktionelle Antikörper, bi- oder oligospezifische Antikörper, einzelsträngige Antikörper, F(ab)- oder F(ab)₂-Fragmente (siehe beispielsweise EP-B1-0 368 684, US 4,816,567, US 4,816,397, WO 88/01649, WO 93/06213, WO 98/24884).

Das Verfahren zur Herstellung von Antikörpern wird nach dem Fachmann allgemein bekannten Methoden durchgeführt, beispielsweise indem ein Säuger, z.B. ein Kaninchen, mit dem oben beschriebenen oder den erwähnten Teilen davon gegebenenfalls in Gegenwart beispielsweise von Freundschem Adjuvans und/oder Aluminiumhydroxidgelen immunisiert wird (siehe beispielsweise Diamond, B.A. et al. (1981) The New England Journal of Medicine, 1344-1349). Die im Tier als Ergebnis einer Immunreaktion gebildeten polyklonalen Antikörper lassen sich dann leicht nach allgemein bekannten Methoden aus dem Blut isolieren und beispielsweise mittels Säulenchromatographie aufreinigen. Monoklonale Antikörper können beispielsweise nach dem bekannten Verfahren von Winter & Milstein (Winter, G. & Milstein, C. (1991) Nature, 349, 293-299) hergestellt werden. Rekombinante Antikörper lassen sich wie in den oben angegebenen Patentveröffentlichungen beschrieben herstellen.

Ein weiterer Gegenstand der vorliegenden Erfindung betrifft ein Verfahren zur Herstellung des EGLN2-Proteins mit einer Aminosäuresequenz gemäß SEQ ID NO: 3, wobei man eine wie oben definierte Zelle in einem geeigneten Kulturmedium kultiviert und gegebenenfalls das EGLN2-Protein aus der Zelle oder dem Kulturmedium isoliert.

Das EGLN2-Protein wird beispielsweise durch Expression- der oben beschriebenen Nukleinsäure in einem geeigneten Expressionssystem, wie bereits oben erläutert wurde, nach den dem Fachmann allgemein bekannten Verfahren hergestellt. Als Wirtszellen eignen sich beispielsweise die *E.coli*-Stämme DHS, HB101 oder BL21, der Hefestamm *Saccharomyces cerevisiae,* die Insektenzellinie Lepidoptera, z.B. von *Spodoptera frugiperda,* oder die tierischen Zellen COS, Vero, 293, HaCaT, und HeLa, die allesamt allgemein erhältlich sind. Das EGLN2-Protein kann auch Teil eines Fusionsproteins sein. Hierbei werden wie oben erläutert Fusionsproteine hergestellt, die die wie oben beschriebenen Polypeptide enthalten, wobei die Fusionsproteine selbst bereits die Funktion eines wie oben beschriebenen Polypeptids aufweisen oder die spezifische Funktion nur nach Abspaltung des Fusionsanteils funktionell aktiv ist. Umfaßt sind hier vor allem Fusionsproteine mit einem Anteil von etwa 1-300, vorzugsweise etwa 1-200, insbesondere etwa 1-100, vor allem etwa 1-50 Fremdaminosäuren. Bei solchen Peptidsequenzen handelt es sich beispielsweise um prokaryontische Peptidsequenzen, die beispielsweise von der Galactosidase aus *E.coli* abgeleitet sein können. Weiterhin lassen sich auch virale Peptidsequenzen, wie beispielsweise des Bakteriophagen M13, verwenden, um so Fusionsproteine für das dem Fachmann bekannte Phagen-Display-Verfahren zu produzieren. Weitere bevorzugte Peptidsequenzen für Fusionsproteine sind beispielsweise Peptide, die einen leichteren Nachweis der Fusionsproteine ermöglichen; dabei handelt es sich beispielsweise um das "Grüne Fluoreszenzprotein" [Green Fluorescent Protein] oder Varianten davon.

Zur Isolierung und/oder Aufreinigung der oben beschriebenen Proteine kann ein weiteres Polypeptid bzw. können weitere Polypeptide (Tag(s)) angefügt werden. Geeignete Protein-Tags gestatten beispielsweise die hochaffine Adsorption an eine Matrix, das stringente Waschen mit geeigneten Puffern, ohne daß dabei der Komplex in einem wahrnehmbaren Ausmaß eluiert wird, und die nachfolgende gezielte Elution des adsorbierten Komplexes. Zu den dem Fachmann bekannten Protein-Tags gehören beispielsweise ein (His)₆-Tag, ein Myc-Tag, ein FLAG-Tag, ein Hämagglutinin-Tag, das Glutathiontransferase (GST)-Tag, Intein mit einem Chitin bindenden Affinitäts-Tag oder das Maltosebindungsprotein (MBP)-Tag. Diese Protein-Tags können N- oder C-terminal und/oder intern lokalisiert sein.

Ein weiterer Gegenstand der vorliegenden Erfindung betrifft ein Verfahren zum Screening eines Modulators eines EGLN2-Proteins, wobei das Verfahren die folgenden Schritte umfaßt:
(a) Bereitstellen des EGLN2-Proteins oder der für das EGLN2-Protein codierenden Nukleinsäure,
(b) Bereitstellen einer Testverbindung und
(c) Messen oder Nachweisen des Einflusses der Testverbindung auf das EGLN2-Protein oder das EGLN2-Gen.

Im allgemeinen wird das EGLN2-Protein oder die für das EGLN2-Protein codierende Nukleinsäure beispielsweise in einem Testsystem bereitgestellt und direkt oder indirekt mit einer Testverbindung, insbesondere einer biochemischen oder chemischen Testverbindung, beispielsweise in Form einer chemischen Verbindungsbibliothek, in Kontakt gebracht. Anschließend wird der Einfluß der Testverbindung auf das EGLN2-Protein oder die für das EGLN2-Protein codierende Nukleinsäure gemessen oder nachgewiesen. Danach kann eine Analyse und/oder Isolierung geeigneter Modulatoren, beispielsweise Aktivatoren oder Inhibitoren, erfolgen. Für das Screening chemischer Verbindungsbibliotheken ist die Verwendung von Tests mit hohem Durchsatz, die dem Fachmann bekannt und kommerziell verfügbar sind, bevorzugt. Gemäß der vorliegenden Erfindung bezieht sich der Begriff "chemische Verbindungsbibliothek" auf mehrere chemische Verbindungen, die aus mehreren beliebigen Quellen, einschließlich chemisch synthetisierter Moleküle und Naturprodukten, zusammengetragen oder die mit Techniken der kombinatorischen Chemie erzeugt wurden.

Im allgemeinen wird der Einfluß der Testverbindung auf EGLN2 oder das EGLN2-Gen in einem heterogenen oder homogenen Test gemessen oder nachgewiesen. Bei einem heterogenen Test, wie er hier verwendet wird, handelt es sich um einen Test, der einen oder mehrere Waschschritte beinhaltet, wohingegen in einem homogenen Test derartige Waschschritte nicht notwendig sind. Die Reagentien und Verbindungen werden lediglich gemischt und gemessen.

Geeignete Funktionstests können auf der Genexpression von EGLN2, der direkten Aktivierung oder Hemmung von EGLN2 beruhen. In Gegenwart einer als Modulator der EGLN2-Genexpression zu testenden biochemischen oder chemischen Verbindung läßt sich die direkte Aktivierung oder Hemmung oder die Komplexbildung mit anderen Proteinen, beispielsweise Zellproteinen, mit dem Fachmann allgemein bekannten Verfahren oder wie bei Appelhoff, R. J. et al. (2004), supra und/oder Jaakkola, P. et al. (2001) Science, 292, 468-472, Nr. 5516 beschrieben messen.

So läßt sich beispielsweise die Prolylhydroxylaseaktivität mittels massenspektrometrischer Analyse, womit die Oxidation von Pro, z.B. Pro⁵⁶⁴ der Prolylhydroxylase oder der HIF-1a -Untereinheit nachgewiesen werden kann, oder mit dem Fachmann bekannten enzymatischen Tests, beispielsweise in einem *In-vitro-*Testverfahren und/oder einem *In*-*vitro*-Testverfahren für ganze Zellen mit menschlichen, tierischen, Bakterien- oder Hefezellen, messen.

Die festphasengebundenen Polypeptide können auch Teil eines Arrays sein. Verfahren zur Herstellung solcher Arrays unter Verwendung der Festphasenchemie und photolabiler Schutzgruppen sind beispielsweise aus der US 5,744,305 bekannt. Diese Arrays können auch mit einer Testverbindung oder Verbindungsbibliotheken in Kontakt gebracht und auf eine Wechselwirkung, beispielsweise Bindung oder Konformationsänderung, getestet werden.

In einer weiteren Ausführungsform der vorliegenden Erfindung wird das Verfahren unter Verwendung ganzer Zellen durchgeführt. Üblicherweise werden am Boden von Multiwell-Platten wachsende Zellen fixiert und permeabilisiert, blockiert und beispielsweise mit einem primären (P)-spezifischen Antikörper gegen das interessierende Substrat inkubiert. Anschließend werden zur Erzeugung des Signals beispielsweise mit Europium markierte oder mit HRP konjugierte sekundäre Antikörper in Verbindung mit spezifischen chemilumineszierenden oder kolorimetrischen Substanzen eingesetzt. In Kombination mit der Verwendung eines Mikroskops läßt sich nicht nur die Menge an (P)-spezifischen Antikörpern auf Einzelzellebene quantifizieren, sondern es können auch durch Phosphorylierung induzierte Translokationen eines Substrats oder morphologische Veränderungen der Zellen quantifiziert werden.

Vorteilhaft wird das Verfahren der vorliegenden Erfindung in einem Robotersystem, das beispielsweise das Ausplattieren mittels Robotern und ein Robotersystem zur Flüssigkeitsübertragung, umfaßt, beispielsweise unter Verwendung von Mikrofluiden, d.h. in einer Kanalstruktur, durchgeführt.

In einer weiteren Ausführungsform der vorliegenden Erfindung wird das Verfahren in Form eines Screeningsystems mit hohem Durchsatz durchgeführt. In einem derartigen System ist das Screening-Verfahren vorteilhafterweise automatisiert und miniaturisiert, wobei insbesondere durch einen Robotor kontrollierte miniaturisierte Vertiefungen (Wells) und Mikrofluide verwendet werden.

Ein weiterer Gegenstand der vorliegenden Erfindung betrifft die Verwendung eines EGLN2-Proteins mit einer Aminosäuresequenz gemäß SEQ ID NO: 2 oder 3 oder einer für das EGLN2-Protein codierenden Nukleinsäure zur Herstellung eines Arzneimittels zur Vorbeugung oder Behandlung thromboembolischer Erkrankungen und/oder koronarer Herzerkrankungen. Bei der thromboembolischen Erkrankung handelt es sich insbesondere um Schlaganfall, prolongiertes reversibles ischämisches neurologisches Defizit (PRIND) und/oder transitorische ischämische Attacke (TIA). Ferner handelt es sich bei der koronaren Herzerkrankung insbesondere um einen Myokardinfarkt. Insbesondere wird das EGLN2-Protein mit einer Aminosäuresequenz gemäß SEQ ID NO: 3 oder eine für das EGLN2-Protein codierende Nukleinsäure für die Herstellung eines Arzneimittels zur Vorbeugung oder Behandlung von Schlaganfall, PRIND und/oder TIA und das EGLN2-Protein mit einer Aminosäuresequenz gemäß SEQ ID NO: 2 oder eine für das EGLN2-Protein codierende Nukleinsäure für die Herstellung eines Arzneimittels zur Vorbeugung oder Behandlung von Myokardinfarkt, insbesondere frühzeitigem Myokardinfarkt, verwendet.

Im einzelnen besteht dabei ein höheres Risiko eines Schlaganfalls, von PRIND und/oder TIA, wenn es sich bei dem Nukleotid in Position 470 um Thymidin in der chromosomalen DNA oder Uracil in der mRNA oder bei der Aminosäure in Position 58 um Leucin handelt. Handelt es sich jedoch bei dem Nukleotid in Position 470 um Cytidin oder bei der Aminosäure in Position 58 um Serin, so besteht ein höheres Risiko eines Myokardinfarkts, insbesondere eines frühzeitigen Myokardinfarkts.

Der Begriff "EGLN2-C470C" bezieht sich gemäß der vorliegenden Erfindung auf diejenige Gruppe von Personen, die auf beiden Allelen des für EGLN2 codierenden Gens in Position 470 der Referenzsequenz NM_053046.2 Cytidin aufweisen, was zur Aminosäure Serin in Position 58 des entsprechenden Proteins führt. Diese Personen sind hinsichtlich dieser EGLN2-Variante homozygot. Folglich bezieht sich der Begriff "EGLN2-C470T" auf diejenige Gruppe von Personen, die auf einem Allel des für EGLN2 codierenden Gens Cytidin aufweisen, was zu Serin in Position 58 des entsprechenden Proteins führt, und auf dem anderen Allel des für EGLN2 codierenden Gens Thymidin aufweisen, was zu Leucin in Position 58 des entsprechenden Proteins führt. Diese Personen sind hinsichtlich dieser EGLN2-Variante heterozygot.

Die Nukleinsäuresequenz der für das menschliche EGLN2-Protein codierenden Referenzsequenz weist vorzugsweise die Nukleinsäuresequenz der SEQ ID NO: 1 und die Aminosäuresequenz des menschlichen EGLN2-Proteins, vorzugsweise die Aminosäuresequenz der SEQ ID NO: 2. auf. Die vorliegende Erfindung umfaßt jedoch auch andere Varianten des menschlichen EGLN2 sowie die nichtmenschlichen Homologen davon, wie beispielsweise andere EGLN2-Homologe aus Säugern oder die EGLN2-Homologe aus *Caenorhabdidis elegans,* Maus oder Ratte, vorausgesetzt, daß in der Position 470 der Referenzsequenz entsprechenden Position ein Nukleotidaustausch von Cytidin zu Thymidin und/oder in der Position 58 der Referenzsequenz entsprechenden Position ein Aminosäureaustausch von Serin zu Leucin vorliegt, und ferner vorausgesetzt, daß das entsprechende Protein eine Prolylhydroxylaseaktivität, insbesondere eine HIF-Prolylhydroxylaseaktivität, aufweist. Die Enzymaktivität läßt sich beispielsweise mittels massenspektrometrischer Analyse messen, wie bereits oben erläutert wurde.

Im allgemeinen läßt sich das spezifische Nukleotid in Position 470 mit einem Nukleinsäuresequenzierungsverfahren, einer massenspektrometrischen Analyse der Nukleinsäure, einem Hybridisierungsverfahren und/oder einem Amplifikationsverfahren bestimmen. Beispiele für ein Nukleinsäuresequenzierungsverfahren sind Pyrosequenzierung und/oder Sequenzierung mit Hilfe von radioaktiven und/oder fluoreszenzmarkierten Nukleotiden. Beispiele für das Hybridisierungsverfahren sind die Southern-Blot-Analyse, die Northern-Blot-Analyse und/oder ein Hybridisierungsverfahren auf einem DNA-Mikroarray. Beispiele für ein Amplifikationsverfahren sind eine TaqMan-Analyse, eine Differential-RNA-Display-Analyse und/oder eine Repräsentationsdifferenzanalyse (Shi M. M. (2002) Am J Pharmacogenomics., 2(3), 197-205; Kozian & Kirschbaum (1999) Trends Biotechnol., 17(2), 73-8.).

Weiterhin läßt sich die Aminosäuresequenz in Position 58 mit einem Verfahren zur Messung der Menge des spezifischen Proteins und/oder einem Verfahren zur Messung der Aktivität des spezifischen Proteins bestimmen. Beispiele für ein Verfahren zur Messung der Menge des spezifischen Proteins sind eine Western-Blot-Analyse und/oder ein ELISA-Test. Beispiele für ein Verfahren zur Messung der Aktivität des spezifischen Proteins sind ein *In*-*vitro*-Testverfahren und/oder ein *In-vitro-*Testverfahren für ganze Zellen mit menschlichen, tierischen, Bakterien- oder Hefezellen, die alle jeweils dem Fachmann bekannt sind.

Bei einer Probe zum Nachweis der jeweiligen Variante handelt es sich beispielsweise um eine Zelle, ein Gewebe oder eine Körperflüssigkeit, insbesondere in zellulären Bestandteilen des Bluts, Endothelzellen oder glatten Muskelzellen. Vorzugsweise wird die Probe zur Isolierung und/oder Aufreinigung der Nukleinsäuren oder von chromosomaler DNA oder der Proteine der Probe für die weitere Analyse mit dem Fachmann bekannten herkömmlichen Verfahren vorbehandelt.

Ein bevorzugtes Verfahren zur Identifizierung einer Variante im Sinne der vorliegenden Erfindung beinhaltet die folgenden Schritte:
(a) Isolieren einer Nukleinsäuresonde, insbesondere einer DNA-Sonde aus einer Probe, insbesondere aus einer Zelle, einem Gewebe, einer Körperflüssigkeit, einem zellulären Bestandteil des Bluts, Endothelzellen oder glatten Muskelzellen, beispielsweise von einer zu untersuchenden Person oder einem zu untersuchenden Patienten;
(b) Amplifizieren des Position 470 des ENGL2-Gens umfassenden spezifischen Bereichs mit Hilfe von Primern, insbesondere der Primer, wie sie in den Beispielen angegeben sind;
(c) Sequenzieren des amplifizierten Bereichs sowie
(d) Analysieren des sequenzierten Bereichs.

Ein alternatives Verfahren zur Bestimmung einer Variante im Sinne der vorliegenden Erfindung beinhaltet die folgenden Schritte:
(a) Isolieren des ENGL2-Proteins aus einer Probe, insbesondere aus einer Zelle, einem Gewebe, einer Körperflüssigkeit, einem zellulären Bestandteil des Bluts, Endothelzellen oder glatten Muskelzellen, beispielsweise von einer zu untersuchenden Person oder einem zu untersuchenden Patienten, und
(b) Bestimmen der Aminosäure in Position 58 des EGLN2-Proteins.

Weitere bevorzugte Schritte sind einzeln oder gemeinsam in den Beispielen angegeben und hiermit durch Bezugnahme auf jeden Schritt aufgenommen.

Mit den folgenden Figuren, Tabellen, Sequenzen und Beispielen soll die vorliegende Erfindung erläutert werden, ohne daß dadurch der Umfang der Erfindung beschränkt wird.

### BESCHREIBUNG DER FIGUREN

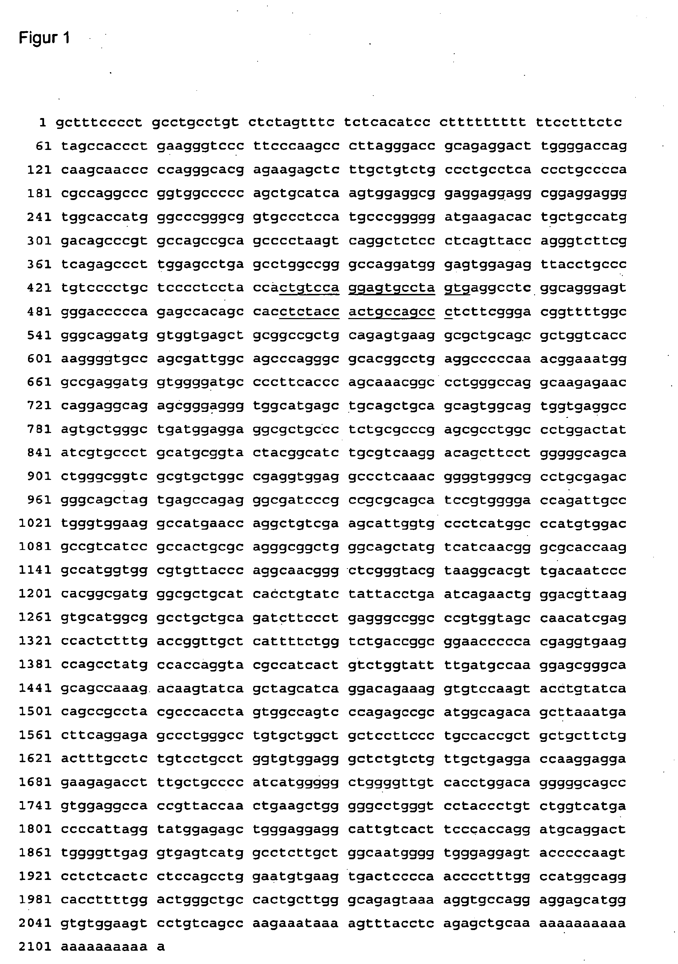

- Figur 1: zeigt die Nukleinsäuresequenz des menschlichen EGLN2-Gens mit der NCBI-Nummer NM_053046. Die zur Amplifikation des Genabschnitts mit der genetischen Variation C→T in Position 470 (in Fettdruck) verwendeten Primer sind unterstrichen.

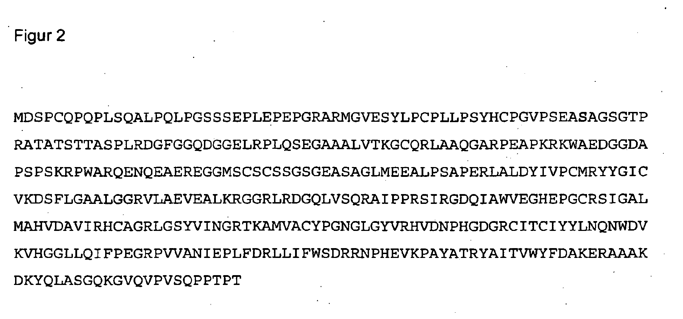

- Figur 2: zeigt die von der Nukleinsäuresequenz mit der NCBI-Nummer NM_053046 abgeleitete Aminosäuresequenz des menschlichen EGLN2. Die Aminosäureposition 58 im EGLN2-Protein ist in Fettdruck dargestellt.

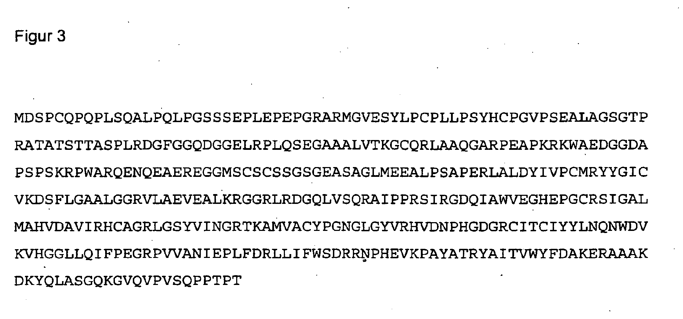

- Figur 3: zeigt die Aminosäuresequenz der menschlichen EGLN2-Variante mit einem Leucin in Position 58. Die Aminosäureposition 58 im EGLN2-Protein ist in Fettdruck dargestellt.

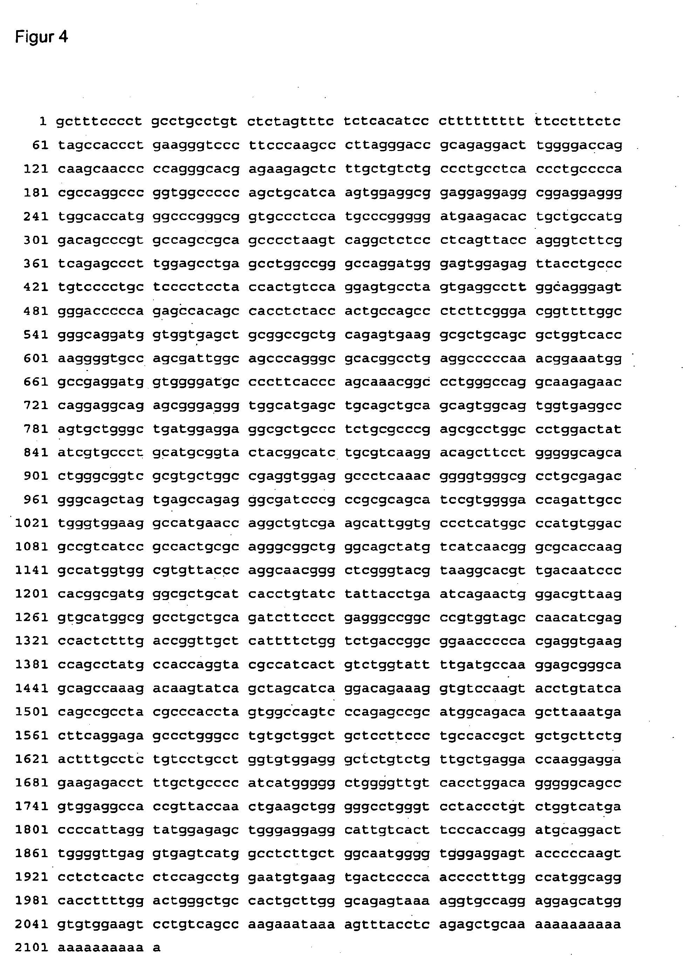

- Figur 4: zeigt die Nukleinsäuresequenz des Gens der menschlichen EGLN2-Variante mit einem Threonin in Position 470.
- Figur 5: zeigt den Einfluß des EGLN2-Genotyps in Position 470 der Referenzsequenz NM_053042.2, der zu Aminosäureaustauschen in Position 58 des EGLN2-Proteins führt, auf das Alter des Auftretens von koronaren Herzerkrankungen in der Patientengruppe. Dabei sind P-Werte unterhalb von 0,05 statistisch relevant.

### BESCHREIBUNG DER SEQUENZEN

SEQ ID NO: 1 zeigt die Nukleinsäuresequenz des menschlichen EGLN2-Proteins mit der NCBI-Nummer NM_053046.
SEQ ID NO: 2 zeigt die von der Nukleinsäuresequenz mit der NCBI-Nummer NM_053046 abgeleitete Aminosäuresequenz des menschlichen EGLN2.
SEQ ID NO: 3 zeigt die Aminosäuresequenz der menschlichen EGLN2-Variante mit einem Leucin in Position 58.
SEQ IN NO: 4 zeigt die Nukleinsäuresequenz des Gens der menschlichen EGLN2-Variante mit einem Threonin in Position 470.
SEQ ID NO 5: zeigt die erste Primersequenz der Nukleotide 444 - 463 der Referenzsequenz NM_053046.2.
SEQ ID NO 6: zeigt die zweite Primersequenz von komplementärer Sequenz der Basen 504 - 521 der Referenzsequenz NM_053046.2.

### BEISPIELE

### SNP-NACHWEIS DURCH SEQUENZIERUNG UND ANALYSE

### Oligonukleotide (Primer) zur Amalifikation:

Zum Nachweis des Nukleotidaustauschs von C zu T in Position 470 in der EGLN2-Sequenz mit der Referenznummer NM_053046.2 wurden die folgenden Primer verwendet:
   Primer 1: 5'- CTGTCCAGGAGTGCCTAGTG -3' (Nukleotide 444 - 463 der Referenzsequenz NM_053046.2; SEQ ID NO: 5);
   Primer 2: 5'- GGGCTGGCAGTGGTAGAG -3' (komplementäre Sequenz der Basen 504 - 521 der Referenzsequenz NM_053046.2; SEQ ID NO: 6).

### PCR-Protokoll zur Amplifikation:

Die verwendeten Reagentien stammten von Applied Biosystems (Foster City, USA):20 ng genomische DNA; 1 Einheit (Unit) TaqGold DNA-Polymerase; 1x Taq-Polymerase-Puffer; 500µM dNTPs; 2,5mM MgCl₂; je 200nM des Amplifikations-Primerpaars, wie oben gezeigt; H₂O ad 5 µl.

### Amplifikationsprogramm der PCR zur Genotypisierung:

| | |
|---|---|
| 95°C,10 min | x 1 Zyklus |
| | |
| 95°C, 30 s | |
| 70°C, 30 s | x 2 Zyklen; |
| | |
| 95°C, 30 s | |
| 65°C, 30 s | x 2 Zyklen; |
| | |
| 95°C, 30 s | |
| 60°C, 30 s | x 2 Zyklen; |
| | |
| 95°C, 30 s | |
| 56°C, 30 s | |
| 72°C, 30 s | x 40 Zyklen; |
| | |
| 72°C, 10 min | |
| 4°C, 30 s | x 1 Zyklus; |

### Protokoll zur Minisequenzierung und zum Nachweis von SNPs

Die verwendeten Reagentien stammten von Applied Biosystems (Foster City, USA). 2 µl gereinigtes PCR-Produkt, 1,5 µl BigDye-Terminator-Kit, 200nM eines Sequenzierprimers, wie oben gezeigt; H₂O ad 10 µl.

### Amplifikationsprogramm zur Sequenzierung:

| | |
|---|---|
| 96°C, 2 min | x 1 Zyklus; |
| | |
| 96°C, 10 s | |
| 55°C, 10 s | |
| 65°C, 4 min | x 30 Zyklen; |
| | |
| 72°C, 7 min | |
| 4°C, 30 s | x 1 Zyklus; |

### Analyse der Sequenzierungsprodukte:

Die Sequenzen wurden zunächst zur Gewinnung vorläufiger Daten mit der Sequenz Analyse Software (Applied Biosystems, Foster City, USA) analysiert und dann mit der Software Phred, Phrap, Polyphred und Consed prozessiert. Phred, Phrap, Polyphred und Consed, geschrieben von Phil Green, Washington University (http://www.genome.washington.edu).

### ERGEBNISSE

### Charakteristika der Gruppe von Personen

Tabelle 1 zeigt die Charakteristika der untersuchten Personengruppe.

**Tabelle 1**

| | | n | % |
|---|---|---|---|
| Gesamt | | 2074 | |
| Geschlecht | Weiblich | 603 | 29,07 |
| | Männlich | 1471 | 70,93 |
| Alter | | 61,8 (+/- 10,5) | |
| BMI (Body Mass Index) | | 29,1 (+/- 4,4) | |
| Blutdruck | | 1214 | 58,7 |
| Raucher | | 1372 | 66,41 |
| Typ-II-Diabetes | | 361 | 17,46 |
| Myokardinfarkt | | 830 | 40,59 |
| Schlaganfall | | 145 | 7,01 |

### Häufigkeit und Verteilung der Varianten des EGLN2-Gens

Tabelle 2 zeigt die Häufigkeit und Verteilung der genetischen Varianten des EGLN2-Gens in Position 470 der Referenzsequenz NM_053046.2 in der untersuchten Patientengruppe.

**Tabelle 2**

| | Häufigkeit | Prozentanteil |
|---|---|---|
| EGNL2-C470C (EGNL2 Ser58Ser) | 1253 | 96,31 |
| EGNL2-C470T (EGNL2 Ser58Leu) | 47 | 3,61 |
| EGNL2-T470T (EGNL2 Leu58Leu) | 1 | 0,08 |
| Fehlende Werte | 773 | |

Im folgenden sind nur Individuen mit EGLN2-C470C (EGLN2 Ser58Ser) und EGLN2-C470T (EGLN2 Ser58Leu) berücksichtigt.

### Einfluß der EGLN2-Varianten auf das Auftreten von frühzeitigem Myokardinfarkt

Tabelle 3 zeigt den Einfluß des EGLN2-Genotyps in Position 470 der Referenzsequenz NM_053046.2 auf das Auftreten von frühzeitigem Myokardinfarkt (bei Männern unter 55 Jahren und bei Frauen unter 60 Jahren) und von Schlaganfall/PRIND (prolongiertes reversibles ischämisches neurologisches Defizit)/TIA (transitorische ischämische Attacke) in der untersuchten Patientengruppe. P-Werte unterhalb von 0,05 sind statistisch relevant.

**Tabelle 3**

| Klinischer Parameter | EGLN2 | | p-Wert |
|---|---|---|---|
| | C470C / Ser58Ser | C470T / Ser58Leu | |
| | n (%) | n (%) | |
| Patienten mit frühzeitigem Myokardinfarkt (<55m/60w) | 215 (17,16) | 2 (4,26%) | 0,0199 |
| Patienten ohne frühzeitigen Myokardinfarkt (<55m/60w) | 1038 (82,84) | 45 (95,74) | |
| Patienten mit Schlaganfall/PRIND/TIA | 88 (7,23) | 7 (14,89) | 0,0418 |
| Patienten ohne Schlaganfall/PRIND/TIA | 1165 (92,77) | 40 (85,11 ) | |

### Ergebnisse:

1. Die Patienten mit EGLN2-C470C zeigten im Vergleich zu Patienten mit EGLN2-C470T eine statistisch höhere Häufigkeit von frühzeitigem Myokardinfarkt.
2. Die Patienten mit EGLN2-C470T zeigten im Vergleich zu Patienten mit EGLN2-C470C einen signifikanten Anstieg des Risikos, einen Schlaganfall, PRIND und/oder TIA zu erleiden.

### Einfluß der EGLN2-Varianten auf das Alter der Patienten mit koronaren Herzerkrankungen

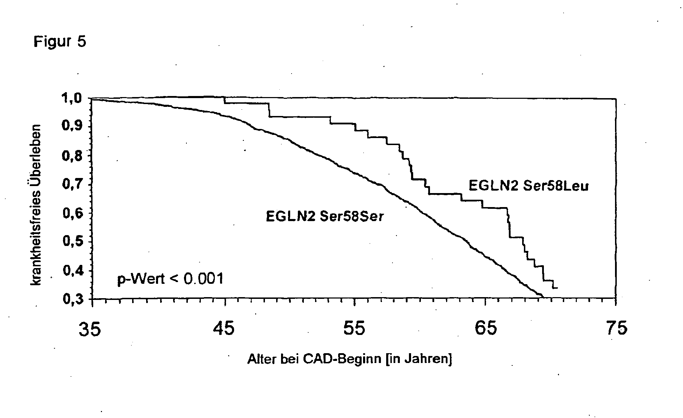

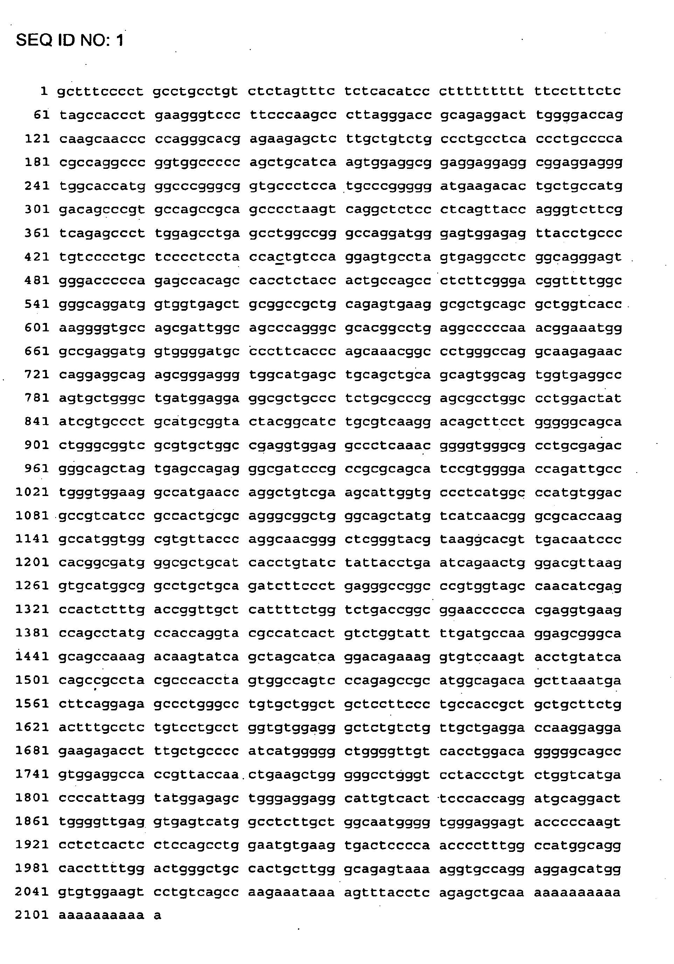

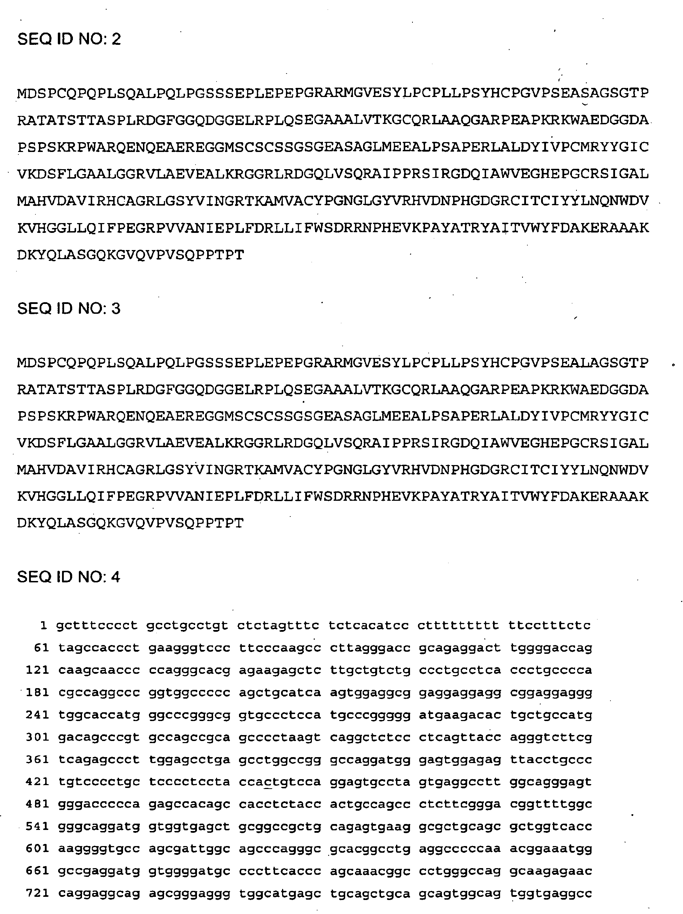

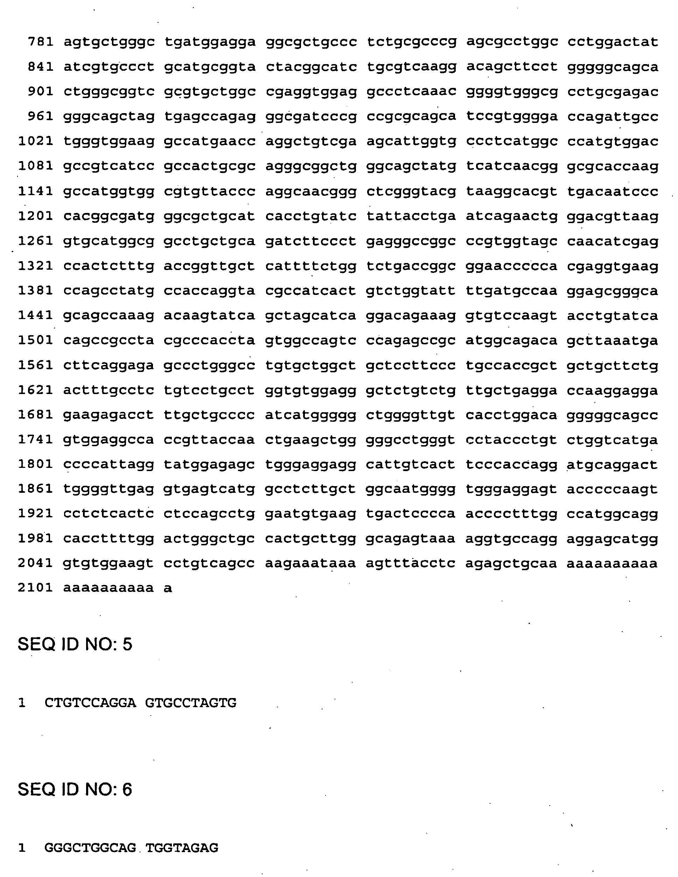

Figur 5 zeigt den Einfluß des EGLN2-Genotyps in Position 470 der Referenzsequenz NM_053042.2 auf das Alter des Auftretens von koronaren Herzerkrankungen in der Patientengruppe.

### Ergebnis:

Es wurde eine signifikante Altersabhängigkeit der Patienten mit EGLN2-C470C (EGLN2 Ser58Ser) für das frühzeitige Auftreten von koronaren Herzerkrankungen im Vergleich zum Alter der Patienten mit EGLN2-C470T (EGLN2 Ser58Leu) festgestellt.

### Schlußfolgerung:

Die oben dargestellten statistisch signifikanten Assoziationen zwischen den genetischen Varianten des für EGLN2 codierenden Gens und/oder des Proteins EGLN2 sind ein deutlicher Hinweis auf die Beteiligung der genetischen Varianten am Auftreten thrombotischer Erkrankungen und/oder koronarer Herzerkrankungen. Folglich stellen die genetischen Varianten biologische Marker für die Prognose thrombotischer Erkrankungen und/oder koronarer Herzerkrankungen, insbesondere für die Prognose von frühzeitigem Myokardinfarkt und/oder Schlaganfall, PRIND und/oder TIA, dar.

## Patentansprüche

1. EGLN2-Protein mit einer Aminosäuresequenz gemäß SEQ ID NO: 3.

2. Nukleinsäure, codierend für das EGLN2-Protein nach Anspruch 1., wobei die Nukleinsäure die Nukleinsäuresequenz gemäß SEQ ID NO: 4. aufweist.

3. Vektor, vorzugsweise Expressionsvektor, mit der Nukleinsäure nach Anspruch 2.

4. Wirtszelle mit einer Nukleinsäure nach Anspruch 2 oder mit einem Vektor nach Anspruch 3.

5. Nicht-humanes transgenes Tier mit einer Nukleinsäure nach Anspruch 2 oder mit einem Vektor nach Anspruch 3.

6. Antikörper mit spezifischer Bindung an das EGLN2-Protein nach Anspruch 1, der im wesentlichen nicht an das EGLN2-Protein mit einer Aminosäuresequenz gemäß seq id no 2 bindet.

7. Verfahren zur Herstellung des EGLN2-Proteins nach Anspruch 1, wobei man eine Zelle nach Anspruch 4 in einem geeigneten Kulturmedium kultiviert und gegebenenfalls das EGLN2-Protein aus der Zelle oder dem Kulturmedium isoliert.

8. Verfahren zum Screening eines Modulators eines EGLN2-Proteins mit einer Aminosäuresequenz gemäß SEQ ID NO: 2 oder 3, wobei das Verfahren die folgenden Schritte umfasst:
(a) Bereitstellen des EGLN2-Proteins oder der für das EGLN2-Protein codierenden Nukleinsäure,
(b) Bereitstellen einer Testverbindung und
(c) Messen oder Nachweisen des Einflusses der Testverbindung auf das EGLN2-Protein oder das EGLN2-Gen.

9. Verfahren nach Anspruch 8, wobei die Testverbindung in Form einer chemischen Verbindungsbibliothek bereitgestellt wird.

10. Verfahren nach Anspruch 8 oder 9, wobei der Einfluss der Testverbindung auf das EGLN2-Protein oder das EGLN2-Gen in einem heterogenen oder homogenen Test gemessen oder nachgewiesen wird.

11. Verfahren nach einem der Ansprüche 8 bis 10, wobei das Verfahren auf einem Array durchgeführt wird.

12. Verfahren nach einem der Ansprüche 8 bis 11, wobei das Verfahren unter Verwendung ganzer Zellen durchgeführt wird.

13. Verfahren nach einem der Ansprüche 8 bis 12, wobei das Verfahren in einem Robotersystem durchgeführt wird.

14. Verfahren nach einem der Ansprüche 8 bis 13, wobei das Verfahren unter Verwendung von Mikrofluiden durchgeführt wird.

15. Verfahren nach einem der Ansprüche 8 bis 14, wobei es sich um ein Screeningverfahren mit hohem Durchsatz handelt.

16. Verwendung eines EGLN2-Proteins mit einer Aminosäuresequenz gemäß SEQ ID NO: 2 oder 3 oder einer für das EGLN2-Protein codierenden Nukleinsäure mit Nukleinsäuresequenz SEQ ID NO: 4 als biologischen Marker für die Prognose thromboembolischer Erkrankungen und/oder koronarer Herzerkrankungen.

17. Verwendung nach Anspruch 16, wobei die thromboembolische Erkrankung ausgewählt ist aus der Gruppe bestehend aus Schlaganfall, prolongiertem reversiblem ischämischem neurologischem Defizit (PRIND) und/oder transitorischer ischämischer Attacke (TIA).

18. Verwendung nach Anspruch 17, wobei es sich bei der koronaren Herzerkrankung um Myokardinfarkt handelt.

19. Verwendung nach Anspruch 17 oder 18, wobei das EGLN2-Protein mit einer Aminosäuresequenz gemäß SEQ ID NO: 3 oder einer für das EGLN2-Protein codierenden Nukleinsäure als biologischer Marker für die Prognose von Schlaganfall, PRIND und/oder TIA verwendet wird.

20. Verwendung nach Anspruch 17 oder 19, wobei das EGLN2-Protein mit einer Aminosäuresequenz gemäß SEQ ID NO: 2 oder einer für das EGLN2-Protein codierenden Nukleinsäure als biologischer Marker für die Prognose von Myokardinfarkt, insbesondere frühzeitigem Myokardinfarkt, verwendet wird.

## Claims

1. EGLN2 protein containing an amino acid sequence according to SEQ ID NO: 3.

2. A nucleic acid coding for the EGLN2 protein according to claim 1, the nucleic acid having the nucleic acid sequence according to SEQ ID NO: 4.

3. A vector, preferably an expression vector, containing the nucleic acid according to claim 2.

4. A host cell containing a nucleic acid according to claim 2 or containing a vector according to claim 3.

5. A non-human transgenic animal containing a nucleic acid according to claim 2 or containing a vector according to claim 3.

6. An antibody specifically binding to the EGLN2 protein according to claim 1, which essentially does not bind to the EGLN2 protein containing an amino acid sequence according to SEQ ID NO. 2.

7. A method for producing the EGLN2 protein according to claim 1, wherein a cell according to claim 4 is cultivated in a suitable culture medium and optionally the EGLN2 protein is isolated from the cell or the culture medium.

8. A method of screening a modulator of an EGLN2 protein containing an amino acid sequence according to SEQ ID NO: 2 or 3, wherein the method comprises the steps of:
(a) providing the EGLN2 protein or the nucleic acid coding for the EGLN2 protein,
(b) providing a test compound, and
(c) measuring or detecting the influence of the test compound on the EGLN2 protein or the EGLN2 gene.

9. The method according to claim 8, wherein the test compound is provided in the form of a chemical compound library.

10. The method according to claim 8 or 9, wherein the influence of the test compound on the EGLN2 protein or the EGLN2 gene is measured or detected in a heterogeneous or homogeneous assay.

11. The method according to any of the claims 8 to 10, wherein the method is carried out on an array.

12. The method according to any of the claims 8 to 11, wherein the method is carried out using whole cells.

13. The method according to any of the claims 8 to 12, wherein the method is carried out in a robotics system.

14. The method according to any of the claims 8 to 13, wherein the method is carried out using microfluidics.

15. The method according to any of the claims 8 to 14, wherein the method is a method of high-throughput screening.

16. Use of an EGLN2 protein containing an amino acid sequence according to SEQ ID NO: 2 or 3 or of a nucleic acid coding for the EGLN2 protein and containing nucleic acid sequence SEQ ID NO: 4 as a biological marker for the prognosis of thromboembolic and/or coronary heart diseases.

17. The use according to claim 16, wherein the thromboembolic disease is selected from the group consisting of stroke, prolonged reversible ischemic neurological deficit (PRIND) and/or transitoric ischemic attack (TIA).

18. The use according to claim 17, wherein the coronary heart disease is myocardial infarction.

19. The use according to claim 17 or 18, wherein the EGLN2 protein containing an amino acid sequence according to SEQ ID NO: 3 or a nucleic acid coding for the EGLN2 protein is used as a biological marker for the prognosis of stroke, PRIND and/or TIA.

20. The use according to claim 17 or 19, wherein the EGLN2 protein containing an amino acid sequence according to SEQ ID NO: 2 or a nucleic acid coding for the EGLN2 protein is used as a biological marker for the prognosis of myocardial infarction, in particular early myocardial infarction.

## Revendications

1. Protéine EGLN2 présentant une séquence d'acides aminés selon la séquence SEQ ID NO : 3.

2. Acide nucléique, codant pour la protéine EGLN2 selon la revendication 1, l'acide nucléique présentant la séquence d'acide nucléique selon la séquence SEQ ID NO : 4.

3. Vecteur, de préférence vecteur d'expression, présentant l'acide nucléique selon la revendication 2.

4. Cellule hôte présentant un acide nucléique selon la revendication 2 ou un vecteur selon la revendication 3.

5. Animal transgénique non humain présentant un acide nucléique selon la revendication 2 ou un vecteur selon la revendication 3.

6. Anticorps présentant une liaison spécifique à la protéine EGLN2 selon la revendication 1, qui ne se lie essentiellement pas à la protéine EGLN2 présentant une séquence d'acides aminés selon la séquence SEQ ID NO:2.

7. Procédé pour la préparation de la protéine EGLN2 selon la revendication 1, une cellule selon la revendication 4 étant cultivée dans un milieu de culture approprié et la protéine EGLN2 étant le cas échéant isolée de la cellule ou du milieu de culture.

8. Procédé de criblage d'un modulateur d'une protéine EGLN2 présentant une séquence d'acides aminés selon la séquence SEQ ID NO : 2 ou 3, le procédé comprenant les étapes suivantes :
(a) mise à disposition de la protéine EGLN2 ou de l'acide nucléique codant pour la protéine EGLN2,
(b) mise disposition d'un composé test et
(c) mesure ou détection de l'influence du composé test sur la protéine EGLN2 ou le gène EGLN2.

9. Procédé selon la revendication 8, le composé test étant mis à disposition sous forme d'une bibliothèque de composés chimiques.

10. Procédé selon la revendication 8 ou 9, l'influence du composé test sur la protéine EGLN2 ou le gène EGLN2 étant mesurée ou détectée dans un test hétérogène ou homogène.

11. Procédé selon l'une quelconque des revendications 8 à 10, le procédé étant réalisé sur une matrice.

12. Procédé selon l'une quelconque des revendications 8 à 11, le procédé étant réalisé avec utilisation de cellules entières.

13. Procédé selon l'une quelconque des revendications 8 à 12, le procédé étant réalisé dans un système de robot.

14. Procédé selon l'une quelconque des revendications 8 à 13, le procédé étant réalisé avec utilisation de microfluides.

15. Procédé selon l'une quelconque des revendications 8 à 14, le procédé étant un procédé de criblage à haut débit.

16. Utilisation d'une protéine EGLN2 présentant une séquence d'acides aminés selon la séquent SEQ ID NO : 2 ou 3 ou d'un acide nucléique codant pour la protéine EGLN2 présentant la séquence d'acide nucléique SEQ ID NO : 4 comme marqueur biologique pour le pronostic de maladies thromboemboliques et/ou de cardiopathies coronariennes.

17. Utilisation selon la revendication 16, la maladie thromboembolique étant choisie dans le groupe constitué par les attaques, le déficit neurologique ischémique réversible prolongé (DNIRP) et/ou un accident ischémique transitoire (AIT).

18. Utilisation selon la revendication 17, la cardiopathie coronarienne étant un infarctus du myocarde.

19. Utilisation selon la revendication 17 ou 18, la protéine EGLN2 présentant une séquence d'acides aminés selon la séquence SEQ ID NO : 3 ou un acide nucléique codant pour la protéine EGLN2 étant utilisé(e) comme marqueur biologique pour le pronostic d'une attaque, d'un DNIRP et/ou d'un AIT.

20. Utilisation selon la revendication 17 ou 19, la protéine EGLN2 présentant une séquence d'acides aminés selon la séquence SEQ ID NO : 2 ou un acide nucléique codant pour la protéine EGLN2 étant utilisé(e) comme marqueur biologique pour le pronostic d'un infarctus du myocarde, en particulier un infarctus du myocarde précoce.


### Sequenz Protokoll

<110> sanofi Aventis Deutschland GmbH
<120> EGLN2-varianten und ihre Verwendung bei der Vorbeugung oder Behandlung thromboembolischer Erkrankungen und koronarer Herzerkrankungen
<130> DE2005/050
<140>
   <141>
<160> 6
<170> PatentIn ver. 2.1
<210> 1
   <212> DNA
   <213> Homo sapiens
<400> 1
<210> 2
   <211> 407
   <212> PRT
   <213> Homo sapiens
<400> 2
   Met Asp Ser Pro Cys Gln Pro Gln Pro Leu Ser Gln Ala Leu Pro Gln
   1               5                   10                  15
   Leu Pro Gly Ser Ser Ser Glu Pro Leu Glu Pro Glu Pro Gly Arg Ala
               20                  25                  30
   Arg Met Gly Val Glu Ser Tyr Leu Pro Cys Pro Leu Leu Pro Ser Tyr
           35                  40                  45
   His Cys Pro Gly Val Pro Ser Glu Ala Ser Ala Gly Ser Gly Thr Pro
       50                  55                  60
   Arg Ala Thr Ala Thr Ser Thr Thr Ala Ser Pro Leu Arg Asp Gly Phe
   65                  70                  75                  80
   Gly Gly Gln Asp Gly Gly Glu Leu Arg Pro Leu Gln Ser Glu Gly Ala
                   85                  90                  95
   Ala Ala Leu Val Thr Lys Gly Cys Gln Arg Leu Ala Ala Gln Gly Ala
               100                 105                 110
   Arg Pro Glu Ala Pro Lys Arg Lys Trp Ala Glu Asp Gly Gly Asp Ala
           115                 120                 125
   Pro Ser Pro Ser Lys Arg Pro Trp Ala Arg Gln Glu Asn Gln Glu Ala
       130                 135                 140
   Glu Arg Glu Gly Gly Met Ser Cys Ser Cys Ser Ser Gly Ser Gly Glu
   145                 150                 155                 160
   Ala Ser Ala Gly Leu Met Glu Glu Ala Leu Pro Ser Ala Pro Glu Arg
                   165                 170                 175
   Leu Ala Leu Asp Tyr Ile Val Pro Cys Met Arg Tyr Tyr Gly Ile Cys
               180                 185                 190
   Val Lys Asp Ser Phe Leu Gly Ala Ala Leu Gly Gly Arg Val Leu Ala
           195                 200                 205
   Glu Val Glu Ala Leu Lys Arg Gly Gly Arg Leu Arg Asp Gly Gln Leu
       210                 215                 220
   Val Ser Gln Arg Ala Ile Pro Pro Arg Ser Ile Arg Gly Asp Gln Ile
   225                 230                 235                 240
   Ala Trp Val Glu Gly His Glu Pro Gly Cys Arg Ser Ile Gly Ala Leu
                   245                 250                 255
   Met Ala His Val Asp Ala Val Ile Arg His Cys Ala Gly Arg Leu Gly
               260                 265                 270
   Ser Tyr Val Ile Asn Gly Arg Thr Lys Ala Met Val Ala Cys Tyr Pro
           275                 280                 285
   Gly Asn Gly Leu Gly Tyr Val Arg His Val Asp Asn Pro His Gly Asp
       290                 295                 300
   Gly Arg Cys Ile Thr Cys Ile Tyr Tyr Leu Asn Gln Asn Trp Asp Val
   305                 310                 315                 320
   Lys Val His Gly Gly Leu Leu Gln Ile Phe Pro Glu Gly Arg Pro Val
                   325                 330                 335
   Val Ala Asn Ile Glu Pro Leu Phe Asp Arg Leu Leu Ile Phe Trp Ser
               340                 345                 350
   Asp Arg Arg Asn Pro His Glu Val Lys Pro Ala Tyr Ala Thr Arg Tyr
           355                 360                 365
   Ala Ile Thr Val Trp Tyr Phe Asp Ala Lys Glu Arg Ala Ala Ala Lys
       370                 375                 380
   Asp Lys Tyr Gln Leu Ala Ser Gly Gln Lys Gly Val Gln Val Pro Val
   385                 390                 395                 400
   Ser Gln Pro Pro Thr Pro Thr
                   405
<210> 3
   <211> 407
   <212> PRT
   <213> Homo sapiens
<400> 3
   Met Asp Ser Pro Cys Gln Pro Gln Pro Leu Ser Gln Ala Leu Pro Gln
   1               5                   10                  15
   Leu Pro Gly Ser Ser Ser Glu Pro Leu Glu Pro Glu Pro Gly Arg Ala
               20                  25                  30
   Arg Met Gly Val Glu Ser Tyr Leu Pro Cys Pro Leu Leu Pro Ser Tyr
           35                  40                  45
   His Cys Pro Gly Val Pro Ser Glu Ala Leu Ala Gly Ser Gly Thr Pro
       50                  55                  60
   Arg Ala Thr Ala Thr Ser Thr Thr Ala Ser Pro Leu Arg Asp Gly Phe
   65                  70                  75                  80
   Gly Gly Gln Asp Gly Gly Glu Leu Arg Pro Leu Gln Ser Glu Gly Ala
                   85                  90                  95
   Ala Ala Leu Val Thr Lys Gly Cys Gln Arg Leu Ala Ala Gln Gly Ala
               100                 105                 110
   Arg Pro Glu Ala Pro Lys Arg Lys Trp Ala Glu Asp Gly Gly Asp Ala
           115                 120                 125
   Pro Ser Pro Ser Lys Arg Pro Trp Ala Arg Gln Glu Asn Gln Glu Ala
       130                 135                 140
   Glu Arg Glu Gly Gly Met Ser Cys Ser Cys Ser Ser Gly Ser Gly Glu
   145                 150                 155                 160
   Ala Ser Ala Gly Leu Met Glu Glu Ala Leu Pro Ser Ala Pro Glu Arg
                   165                 170                 175
   Leu Ala Leu Asp Tyr Ile Val Pro Cys Met Arg Tyr Tyr Gly Ile Cys
               180                 185                 190
   Val Lys Asp Ser Phe Leu Gly Ala Ala Leu Gly Gly Arg Val Leu Ala
           195                 200                 205
   Glu Val Glu Ala Leu Lys Arg Gly Gly Arg Leu Arg Asp Gly Gln Leu
       210                 215                 220
   Val Ser Gln Arg Ala Ile Pro Pro Arg Ser Ile Arg Gly Asp Gln Ile
   225                 230                 235                 240
   Ala Trp Val Glu Gly His Glu Pro Gly Cys Arg Ser Ile Gly Ala Leu
                   245                 250                 255
   Met Ala His Val Asp Ala Val Ile Arg His Cys Ala Gly Arg Leu Gly
               260                 265                 270
   Ser Tyr Val Ile Asn Gly Arg Thr Lys Ala Met Val Ala Cys Tyr Pro
           275                 280                 285
   Gly Asn Gly Leu Gly Tyr Val Arg His Val Asp Asn Pro His Gly Asp
       290                 295                 300
   Gly Arg Cys Ile Thr Cys Ile Tyr Tyr Leu Asn Gln Asn Trp Asp Val
   305                 310                 315                 320
   Lys Val His Gly Gly Leu Leu Gln Ile Phe Pro Glu Gly Arg Pro Val
                   325                 330                 335
   Val Ala Asn Ile Glu Pro Leu Phe Asp Arg Leu Leu Ile Phe Trp Ser
               340                 345                 350
   Asp Arg Arg Asn Pro His Glu Val Lys Pro Ala Tyr Ala Thr Arg Tyr
           355                 360                 365
   Ala Ile Thr Val Trp Tyr Phe Asp Ala Lys Glu Arg Ala Ala Ala Lys
       370                 375                 380
   Asp Lys Tyr Gln Leu Ala Ser Gly Gln Lys Gly Val Gln Val Pro Val
   385                 390                 395                 400
   Ser Gln Pro Pro Thr Pro Thr
                   405
<210> 4
   <211> 2111
   <212> DNA
   <213> Homo sapiens
<400> 4
   gctttcccct gcctgcctgt ctctagtttc tctcacatcc cttttttttt ttcctttctc 60
   tagccaccct gaagggtccc ttcccaagcc cttagggacc gcagaggact tggggaccag 120
   caagcaaccc ccagggcacg agaagagctc ttgctgtctg ccctgcctca ccctgcccca 180
   cgccaggccc ggtggccccc agctgcatca agtggaggcg gaggaggagg cggaggaggg 240
   tggcaccatg ggcccgggcg gtgccctcca tgcccggggg atgaagacac tgctgccatg 300
   gacagcccgt gccagccgca gcccctaagt caggctctcc ctcagttacc agggtcttcg 360
   tcagagccct tggagcctga gcctggccgg gccaggatgg gagtggagag ttacctgccc 420
   tgtcccctgc tcccctccta ccactgtcca ggagtgccta gtgaggcctt ggcagggagt 480
   gggaccccca gagccacagc cacctctacc actgccagcc ctcttcggga cggttttggc 540
   gggcaggatg gtggtgagct gcggccgctg cagagtgaag gcgctgcagc gctggtcacc 600
   aaggggtgcc agcgattggc agcccagggc gcacggcctg aggcccccaa acggaaatgg 660
   gccgaggatg gtggggatgc cccttcaccc agcaaacggc cctgggccag gcaagagaac 720
   caggaggcag agcgggaggg tggcatgagc tgcagctgca gcagtggcag tggtgaggcc 780
   agtgctgggc tgatggagga ggcgctgccc tctgcgcccg agcgcctggc cctggactat 840
   atcgtgccct gcatgcggta ctacggcatc tgcgtcaagg acagcttcct gggggcagca 900
   ctgggcggtc gcgtgctggc cgaggtggag gccctcaaac ggggtgggcg cctgcgagac 960
   gggcagctag tgagccagag ggcgatcccg ccgcgcagca tccgtgggga ccagattgcc 1020
   tgggtggaag gccatgaacc aggctgtcga agcattggtg ccctcatggc ccatgtggac 1080
   gccgtcatcc gccactgcgc agggcggctg ggcagctatg tcatcaacgg gcgcaccaag 1140
   gccatggtgg cgtgttaccc aggcaacggg ctcgggtacg taaggcacgt tgacaatccc 1200
   cacggcgatg ggcgctgcat cacctgtatc tattacctga atcagaactg ggacgttaag 1260
   gtgcatggcg gcctgctgca gatcttccct gagggccggc ccgtggtagc caacatcgag 1320
   ccactctttg accggttgct cattttctgg tctgaccggc ggaaccccca cgaggtgaag 1380
   ccagcctatg ccaccaggta cgccatcact gtctggtatt ttgatgccaa ggagcgggca 1440
   gcagccaaag acaagtatca gctagcatca ggacagaaag gtgtccaagt acctgtatca 1500
   cagccgccta cgcccaccta gtggccagtc ccagagccgc atggcagaca gcttaaatga 1560
   cttcaggaga gccctgggcc tgtgctggct gctccttccc tgccaccgct gctgcttctg 1620
   actttgcctc tgtcctgcct ggtgtggagg gctctgtctg ttgctgagga ccaaggagga 1680
   gaagagacct ttgctgcccc atcatggggg ctggggttgt cacctggaca gggggcagcc 1740
   gtggaggcca ccgttaccaa ctgaagctgg gggcctgggt cctaccctgt ctggtcatga 1800
   ccccattagg tatggagagc tgggaggagg cattgtcact tcccaccagg atgcaggact 1860
   tggggttgag gtgagtcatg gcctcttgct ggcaatgggg tgggaggagt acccccaagt 1920
   cctctcactc ctccagcctg gaatgtgaag tgactcccca accccttttg ccatggcagg 1980
   caccttttgg actgggctgc cactgcttgg gcagagtaaa aggtgccagg aggagcatgg 2040
   gtgtggaagt cctgtcagcc aagaaataaa agtttacctc agagctgcaa aaaaaaaaaa 2100
   aaaaaaaaaa a                                                      2111
<210> 5
   <211> 20
   <212> DNA
   <213> Künstliche sequenz
<220>
   <223> Beschreibung der künstlichen Sequenz: Primer
<400> 5
   ctgtccagga gtgcctagtg 20
<210> 6
   <211> 18
   <212> DNA
   <213> Künstliche sequenz
<220>
   <223> Beschreibung der künstlichen Sequenz: Primer
<400> 6
   gggctggcag tggtagag 18